# EUROPEAN PATENT SPECIFICATION

(11) **EP 4 139 213 B1**
(45) Date of publication and mention of the grant of the patent: **08.07.2026**
(21) Application number: 21711657.3
(22) Date of filing: 26.02.2021
(51) Int. Cl.: B65B 31/02, A61K 8/00, A61Q 19/00

(54) **COSMETIC FORMULATIONS WITH AN ARGON BLANKET AND AN INTERNAL ARGON RESERVOIR AND METHODS FOR MAKING SAME**
KOSMETISCHE FORMULIERUNGEN MIT EINER ARGONDECKE UND EINEM INTERNEN ARGONRESERVOIR SOWIE HERSTELLUNGSVERFAHREN DAFÜR
FORMULATIONS COSMÉTIQUES À COUCHE D'ARGON ET RÉSERVOIR D'ARGON INTERNE ET PROCÉDÉS DE FABRICATION DE TELLES FORMULATIONS

(30) Priority: 28.02.2020 IS 9133
(43) Date of publication of application: 01.03.2023
(73) Proprietor: TARAMAR SEEDS EHF., 245 Sudurnesjabaer (IS)
(72) Inventor: MARTEINSDOTTIR, Gudrun, 111 Reykjavik (IS); KRISTBERGSSON, Kristberg, 111 Reykjavik (IS); KRISTJANSSON, Jakob, 104 Reykjavik (IS)
(74) Representative: Arnason Faktor
(86) International application number: PCT/IS2021/050004
(87) International publication number: WO 2021/171321

(56) References cited:
- EP-A2- 1 192 939
- WO-A1-2008/009617
- WO-A2-2010/001049
- US-A1- 2004 215 066

## Description

### Field of the Invention

This disclosure generally relates to systems and methods for preserving organic formulations. More specifically, the present disclosure relates to systems and methods for preserving organic formulations using argon-gas-infused discrete compartments such as microbubbles.

### Technical Background and Prior Art

Consumers use various organic formulations in their everyday life. However, nearly all unpreserved organic formulations are susceptible to spoilage owing to the naturally high content of water and organic compounds found within these products that can serve as a food source for contaminating microbes. As a result, manufacturers of organic formulations routinely use chemical preservatives to prevent microbial growth and spoilage of these products. Unfortunately, many chemical preservatives are being linked to unintended negative effects on the health or well-being of consumers.

Cosmetic formulations are a subset of organic formulations, and many consumers use cosmetic formulations on a daily basis to care for and beautify their skin and hair. Just like organic formulations, generally, cosmetic formulations suffer from a susceptibility to spoilage due to microbial contamination. Microbes, particularly fungi, tend to thrive within the moist, nutrient rich medium of these products. In addition to microbial contamination, cosmetic formulations are additionally subject to spoilage that results from oxidation of active ingredients. Consequently, most manufacturers include chemical (synthetic) preservatives to prevent spoilage of the cosmetic, whether from microbial growth or due to oxidation or other chemical degradation affecting the efficacy of the cosmetic formulation.

Recent evidence has shown, however, that the use of chemical preservatives in cosmetic formulations has had some unintended consequences. For example, different types of parabens that have commonly been used as chemical preservatives in cosmetic formulations have been linked with cancer (Halla et. al. 2018). Other commonly used chemical preservatives have been similarly linked with dire side effects, and even some of the milder preservatives that are believed to be safer alternatives are now in question.

Phenoxyethanol for example, is used by many manufacturers as a chemical preservative in lieu of parabens, and while phenoxyethanol is currently accepted by many regulatory agencies, its safety in cosmetic formulations is questionable. The U.S. Federal Drug Administration has issued a warning for its use in nipple creams, and Japan restricts its use in cosmetic formulations to concentrations less than 1% owing, at least in part, to its ease of absorption through the skin and potential negative side effects associated therewith. Despite this chemical's restriction in both concentration and location of application in many countries, it is frequently found in cosmetic formulations at concentrations above the restricted limits (e.g., up to 15%). This is particularly troubling given that phenoxyethanol has been shown to accumulate to detectable levels in live cells, even after processing within the kidneys. The exact impact of prolonged and repeated exposure to phenoxyethanol has not yet been elucidated, but many consumers are unwilling to risk the potentially negative consequences of using consumer care products that include this, or other chemical preservatives, particularly given the toxicological effects and questionable past of chemical preservatives within cosmetic formulations.

Consumers are increasingly avoiding products that include the foregoing and other chemical preservatives and are, instead, favouring cosmetic formulations that are non-toxic and made from certified ingredients. According to consumer surveys, as many as 55% of consumers read the labels of cosmetic formulations prior to purchase and do so specifically to avoid certain ingredients and chemical preservatives. This behaviour is particularly prominent among consumers with children at home. Surveys report that consumers with children in the home are 69% more likely to choose "green" cosmetic formulations (e.g., products that are non-toxic and environmentally safe) compared to consumers without children in the home. In the search for non-toxic products, consumers are increasingly directed towards certified-organic products, which do not contain chemical preservatives.

As expected, however, organic cosmetic formulations tend to have a shorter shelf life than similar products that include chemical preservatives. Organic cosmetic formulations have a much higher likelihood of spoiling due to unwanted microbial growth and can also lose their freshness or efficacy more quickly. For example, in the absence of chemical preservatives, active ingredients may oxidize more quickly, rendering them less efficacious or entirely worthless for their intended purpose. Some natural preservatives may be incorporated into organic products, however according to our experience, natural preservatives on their own cannot be trusted to preserve organic formulations 100%. In some instances, natural preservatives when used alone fail both to preserve the cosmetic formulation (e.g., prevent oxidation of the active ingredients) and to prevent spoilage from microbial contamination (e.g., prevent the growth or survival of microorganisms on the product). When cosmetic formulations spoil from microbial contamination, the microbes are typically growing on the surface of the cosmetic formulation or within the headspace of the container. When natural preservatives are used as the only preservation method, they typically fail to prevent microbial growth in the headspace of containers. In contrast to synthetic preservatives that evaporate and create a toxic atmosphere in the container's headspace, natural compounds rarely evaporate and therefore have little influence outside the formulated medium. As a result, beneficial conditions for microbial growth, particularly fungal growth, are frequently created in the headspace of containers housing organic cosmetic formulations.

WO 2010/001049 discloses an approach to minimise contact of a cosmetic or pharmaceutical formulation with the air, by using a container with a leak-tight dispenser, such as pump or a valve, and (ii) by forming a headspace with an inert gas, or alternatively having a vacuum in the headspace after having filled the space with an inert gas (referred to as "inert gas vacuum"). For this purpose, a fluid-packaging device is provided, where the filling of a cosmetic container takes place in a special chamber that can be evacuated and then filled with inert gas, the chamber being fluidly connected with an inert gas source (G) and a vacuum source (V) (pump). The document mentions that the inert gas may be nitrogen or argon.

WO 2008/009617 is concerned with foams having a controlled fine air bubble size distribution and to edible products prepared therefrom having a low fat content, in particular ice creams and related frozen products.

EP 1192939 A2 relates to compositions with certain active ingredients and in one example, argon is bubbled through water being used to purge oxygen out (these bubbles do not remain in the wate, the argon is simply used for gassing-out the water), and the composition is then formulated under argon, presumably because some of the ingredients are sensitive to oxygen.

Similarly, US 2004/215066 A1 describes preparation of a particular formulation free from oxygen by purging argon gas through water prior to use and blanketing lipid and water phases prior to mixing.

Prior art methods disclose the use of inert gas blankets for preserving cosmetics. For example, U.S. Patent Publication No. 2011/0146207 to Stéphane Desrues discloses methods for packaging formulations, including cosmetics, with a vacuum seal. In particular, the methods disclosed in the Desrues publication include filling containers with a cosmetic formulation in an inert gas environment, pulling a vacuum from the inert gas environment containing the cosmetic filled container and sealing the container in the inert-gas vacuum. As suggested by the Desrues publication, the resulting container has increased storage stability because any remaining "air" inside of the container after it has been sealed is an inert gas that cannot oxidize any organic compounds in the container.

As an additional example, German Patent No. DE 3912153 to Christoph Stein discloses the use of argon to reduce the dissolved oxygen concentration in liquid cosmetic solutions and to replace air from the headspace of the associated containers. Notably, this reference utilized argon in a 5 - 80% mixture with nitrogen gas and discloses CO₂ as a superior gas to argon at reducing the dissolved oxygen. As suggested by the Stein patent, forcing the argon-nitrogen mixture or CO₂ through a liquid cosmetic can reduce the dissolved oxygen concentration and aid in the preservation of the cosmetic.

Accordingly, there are a number of problems in the art of preserving cosmetic formulations that can be addressed.

### Summary of the invention

The invention is defined in the appended claims. Embodiments of the present invention solve one or more of the foregoing or other problems in the art with preserving cosmetic formulations. In particular, one or more embodiments can include a system for preserving a cosmetic formulation of the invention and for preventing growth or survival of microorganisms on the cosmetic formulation. The system includes a cosmetic formulation container and the cosmetic formulation disposed within the cosmetic formulation container. The cosmetic formulation includes an argon gas reservoir that is formed within the cosmetic formulation such that rupturing the argon gas reservoir releases argon gas onto an adjacent cosmetic-air interface, displacing oxygenated air from at the adjacent cosmetic-air interface.

The cosmetic formulation contains natural preservatives and an argon gas reservoir that is formed within the cosmetic formulation such that rupturing the argon gas reservoir releases argon gas onto an adjacent cosmetic-air interface, displacing oxygenated air from at the adjacent cosmetic-air interface. The term "cosmetic-air interface" as used herein refers to the interface between a cosmetic formulation and air or gas, such as the visible surface of a formulation when a container with the formulation is opened, i.e. the cosmetic-air interface is the interface between the formulation surface and the headspace underneath the lid or other closure.

The system additionally includes a closure such as typically a lid securely attached to the cosmetic formulation container to close the container, and a headspace defined between the lid and the cosmetic-air interface of the cosmetic formulation. The headspace can be at least partially filled with argon gas to form an argon gas blanket that displaces oxygenated air from at least a portion of the cosmetic-air interface. The argon gas reservoir can be configured to replenish the argon gas blanket when the argon gas blanket is disrupted during use or extraction of the cosmetic formulation from the cosmetic formulation container.

The argon gas reservoir is defined by a plurality of microbubbles that are dispersed throughout the cosmetic formulation. The microbubbles can be uniform in size or non-uniform in size. In either instance, the microbubbles can in some embodiments have an average diameter less than about 1 mm, preferably between about 10 µm - 1 mm, or more preferably between about 50 µm - 500 µm and are preferably uniformly distributed within the cosmetic formulation.

In some embodiments, the cosmetic formulation is selected from a cream, gel, mousse, lotion, paste, foam, ointment, suspension, and emulsion.

An aspect of the invention provides a process for process for preparing and preserving a cosmetic formulation that is substantially without synthetic preservatives, the process comprising the steps of preparing a cosmetic formulation, substantially without synthetic preservatives, filling a container with argon gas, forming an argon gas reservoir within the cosmetic formulation confined in compartments within the cosmetic formulation comprising plurality of bubbles, introducing the cosmetic formulation and argon gas reservoir into said container, thereby displacing at least part of said argon gas occupying said container, providing argon gas to the headspace above the cosmetic formulation to form an argon gas blanket, and sealing the container with a closure. The formulation produced with the process is a formulation according to the invention, as further described herein, that comprises natural preservatives. The closure is in some embodiments a lid but other embodiments include but are not limited to hand pumps, e.g. screw-on pumps, or other types of dispensers suitable for the cosmetic formulation.

Embodiments of the present disclosure additionally include methods of manufacturing and preserving a cosmetic formulation. An exemplary method can include at least the steps of (i) preparing a cosmetic formulation that is free of exogenous chemical preservatives, (ii) forming an argon gas reservoir within the cosmetic formulation, wherein the argon gas reservoir is formed such that rupturing the argon gas reservoir releases argon gas onto a cosmetic-air interface, displacing oxygenated air from the cosmetic-air interface, and (iii) sealing a cosmetic formulation container comprising the cosmetic formulation in a nonvacuum, argon gas environment.

Embodiments of the present disclosure additionally include methods of manufacturing and preserving a cosmetic formulation. An exemplary method can include at least the steps of (i) preparing a cosmetic formulation that contains natural preservatives, (ii) forming an argon gas reservoir within the cosmetic formulation, wherein the argon gas reservoir is formed such that rupturing the argon gas reservoir releases argon gas onto a cosmetic-air interface, displacing oxygenated air from the cosmetic-air interface, and (iii) sealing a cosmetic formulation container comprising the cosmetic formulation in a nonvacuum, argon gas environment.

In some embodiments, forming the argon gas reservoir includes infusing a plurality of microbubbles within the cosmetic formulation with argon gas. The microbubbles can have an average diameter of less than 1 µm, preferably between about 10 µm - 1 mm, and more preferably between about 50 µm - 500 µm, and in some manufacturing methods, the microbubbles are formed by stirring a gel forming compound into the prepared cosmetic formulation, preferably within an argon gas environment. In some embodiments the argon reservoir comprises additionally or alternatively larger bubbles, such as bubbles with a diameter in the range of 1 to 10 mm.

As provided above, methods disclosed herein can include the step of sealing the cosmetic formulation container in a nonvacuum, argon gas environment. Nonvacuum environment is rare or not known in cosmetic production, where the general method in producing cosmetic formula is performed in a vacuum environment. In some methods said sealing causes a headspace of the cosmetic formulation container to be at least partially filled with argon.

Embodiments of the present disclosure can additionally include cosmetic formulations, such as those made by any of the disclosed methods and process. It follows that the formulations of the invention are substantially without conventional synthetic preservatives but containing natural preservatives and having an argon gas reservoir formed within the cosmetic formulation for preserving the cosmetic formulation. The argon gas reservoir is suitably configured as is described herein, such as by microbubbles as described herein. The cosmetic formulation can additionally be made with and include certified-organic ingredients.

In some embodiments the formulation is selected from a cream, gel, mousse, lotion, paste, foam, ointment, suspension, and emulsion.

The argon reservoir may include a plurality of microbubbles dispersed throughout the cosmetic formulation. The microbubbles can have an average diameter less than about I mm ,preferably between about 10 µm - 1 mm, or more preferably between about 50 µm - 500 µm. The microbubbles can be substantially uniform in size and/or be substantially uniformly distributed throughout the cosmetic formulation.

Accordingly, systems and methods for preserving a cosmetic formulation, and compositions included within said systems or made by said methods are disclosed.

This summary is provided to introduce a selection of concepts in a simplified form that are further described below in the detailed description. This summary is not intended to identify key features or essential features of the claimed subject matter, nor is it intended to be used as an indication of the scope of the claimed subject matter.

Additional features and advantages of the disclosure will be set forth in the description which follows, and in part will be obvious from the description, or may be learned by the practice of the disclosure. The features and advantages of the disclosure may be realized and obtained by means of the instruments and combinations particularly pointed out in the appended claims. These and other features of the present disclosure will become more fully apparent from the following description and appended claims or may be learned by the practice of the disclosure as set forth hereinafter.

### Brief description of the drawings

In order to describe the manner in which the above recited and other advantages and features of the disclosure can be obtained, a more particular description of the disclosure briefly described above will be rendered by reference to specific embodiments thereof, which are illustrated in the appended drawings. It is appreciated that these drawings depict only typical embodiments of the disclosure and are not therefore to be considered to be limiting of its scope. The disclosure will be described and explained with additional specificity and detail through the use of the accompanying drawings in which.
Figure 1 is a schematic view of an embodiment of a packaged cosmetic formulation.
Figure 2 is a schematic view of another embodiment of a packaged cosmetic formulation.
Figure 3A is a detail view of an embodiment of the cosmetic formulation from section A of Figure 2.
Figure 3B is a detail view of another embodiment of the cosmetic formulation from section A of Figure 2.
Figure 4 is a schematic view of another embodiment of a packaged cosmetic formulation.
Figure 5 is an exemplary method of manufacturing and preserving a cosmetic formulation.

### DETAILED DESCRIPTION OF THE INVENTION

While the disclosure is susceptible to various modifications and alternative constructions, certain illustrative embodiments are in the drawings and are described below. It should be understood, however, there is no intention to limit the disclosure to the specific embodiments disclosed, but on the contrary, the intention covers all modifications, alternative constructions, combinations, and equivalents falling within the scope of the claims.

As used herein, the term "organic formulation" includes any composition having at least one component with a carbon base. This can include for example, cosmetic formulations, food products, and petroleum products or its organic derivatives.

The term "cosmetic formulation," as used herein, includes any personal care product or formulation, whether in a solid, liquid, gel, cream, or other textural medium, that can be used to care for, improve, or beautify the condition or appearance of a person's hair and/or skin. The term "cosmetic formulation" is intended to include skin care products (e.g., balms, lotions, creams, cleansers, exfoliators, peels, scrubs, face wash, moisturizers, face masks, etc.), hair care products (e.g., shampoos, conditioners, hair styling gels, hairsprays, mousse, etc.), makeup, and other cosmetics.

In some instances, a cosmetic formulation includes certified-organic ingredients. As used herein, the term "certified-organic" is meant to include those ingredients that are free of synthetic additives like pesticides, chemical fertilizers and dyes. Further, a "certified-organic" ingredient is one that is not processed using industrial solvents or irradiation and is not the product of genetic engineering. When the term "certified-organic" is used to describe a cosmetic formulation, the term is understood to encompass those cosmetic formulations that include an ingredients list with at least 95% of the ingredients being "certified-organic" ingredients. Preferably "certified organic" indicates that the respective substance or product meets the criteria for organic labelled cosmetics as set forth by USDA and FDA. (See for example FDA's website at https://www.fda.gov/cosmetics/cosmetics-labeling-claims/organic-cosmetics.)

In some instances, a cosmetic formulation includes natural preservatives. As used herein, the term "natural preservative" is meant to include natural products that have antimicrobial properties that are free of synthetic chemicals like parabens and phenoxyethanol. Further, a "natural preservative" is one that is produced by purely biological process or is extracted from various herbs, such as rosemary, clove, thyme, cinnamon, tea tree and lavender or various vegetables such as cabbage and radishes, and are not processed using industrial solvents or irradiation and is not the product of genetic engineering. When the term "natural preservative" is used to describe a cosmetic formulation, the term is understood to encompass those cosmetic formulations that include a low amount of such natural or purely biological ingredient with antimicrobial properties.

### Overview

As described above, there is an unsolved problem in the art of preserving organic formulations, especially cosmetic formulations, in such a way that it does not compromise the health of users. Without exogenous chemical preservatives, organic formulations have a decreased shelf life due to spoilage from microbial contamination and/or from oxidation of the active ingredients. Natural preservatives (i.e., preservatives extracted directly from plant life and not chemically synthesized in a laboratory or commercial setting) and when used alone, have thus far failed to preserve organic formulations in a commercially viable way.

The present disclosure provides systems, compositions, and methods for preserving organic formulations and/or for preventing the growth or survival of microorganisms on organic formulations that solve one or more problems in the art. In particular, the systems, compositions, and methods disclosed herein include the use of argon gas to preserve cosmetic formulations. In a preferred embodiment, the systems, compositions, and methods are directed to preserving cosmetic formulations using an argon gas blanket and an argon gas reservoir for replenishing the argon gas blanket during use.

The systems, compositions, and methods are directed to preserving cosmetic formulations by using together a natural preservative in a composition and an argon gas blanket and an argon gas reservoir for replenishing the argon gas blanket during use.

The use of inert gas blankets, generally, for the preservation of organic formulations, even cosmetic formulations, is known in the art (Clum et. al. 1993; Gurge and Trumbore, 2009). Inert gas blankets remove oxygenated air from contact with the cosmetic formulation, which can beneficially serve a two-fold purpose.

First, the lack of oxygenated-air at the cosmetic-air interface precludes any further oxidation of ingredients or compounds within the associated formulation - at least until the inert gas blanket is disrupted or removed.

Second, the inert gas blanket creates an anaerobic environment within the headspace of the container and particularly at the cosmetic-air interface. As the overwhelming majority of microbes, especially fungi, that feed upon cosmetic formulations are aerobic in nature this prevents growth of these microbes on the cosmetic formulation, and in some instances, the anaerobic environment created by the gas blanket can additionally kill microbes within the headspace of the container.

Prior art documents Desrues and Stein discussed in the Background Section fail to preserve an organic formulation, namely cosmetic formulations, after the associated container is opened. That is, the non-oxidizing "inert gases" disclosed in the prior art diffuse away from the cosmetic-air interface when the container is opened, exposing the cosmetic-air interface to oxygenated air that can lead to spoilage.

An exception to this is argon. Because argon is "heavier" (i.e., denser) than atmospheric air, applying argon gas to the headspace of cosmetic formulation containers causes atmospheric air to be displaced and creates an argon gas blanket over the cosmetic-air interface that resists diffusion into atmospheric air upon opening of the container.

However, the Desrues publication, like many of the prior art preservation techniques, is agnostic to the type of inert gas used, insofar as the "inert gas does not react with the fluid in such a manner as to spoil it" (see the Desrues publication). The preservation methods described in the prior art, which are typified by Desrues, include argon within a laundry list of non-oxidizing, "inert gases," such as nitrogen, carbon dioxide, helium, or argon but fail to preferentially select argon or distinguish it from the other "inert gases." Instead, argon is treated as mostly interchangeable with the other "inert gases," and if anything, prior art methods disfavour argon because it is generally more expensive to implement than other inert gases, such as carbon dioxide and nitrogen.

Problematically, even if an argon gas blanket is used according to the prior art methods, any originally deposited argon gas in the headspace of the container can be disrupted, thinned, or depleted as the user removes cosmetic from the container, limiting its utility as a preservative.

For example, after opening a cosmetic formulation container having an argon blanket associated at the cosmetic-air interface, the user can, by a swiping motion, remove cosmetic from the container. In doing so, the argon gas blanket will be displaced by the user's finger and/or by the turbulence of the swiping motion, which can cause some of the argon gas blanket to spill over the side of the container and out of the headspace - thereby reducing the total volume of argon within the headspace for forming an argon gas blanket over the cosmetic-air interface.

In addition to the volume reduction of argon gas in the headspace, removal of a portion of the cosmetic formulation causes an increase in the surface area of the cosmetic-air interface and a decrease in the depth of the argon gas blanket over undisrupted surfaces of the cosmetic formulation. In other words, removal of a portion of the cosmetic formulation can result in the formation of a divot in the cosmetic, and the argon gas blanket will fill the volume of the divot and spread over the additional surface area related thereto. Without a means of replenishing the argon gas blanket, the cosmetic-air interface will thin over the remaining cosmetic-air interface, and repeated use will eventually cause portions of the cosmetic-air interface to be continually exposed to atmospheric, oxygenated air, allowing oxidation of the active ingredients and/or allowing microbial contamination and spoilage of the cosmetic.

The foregoing and other problems in the art of preserving organic formulations remain unsolved by prior art methods and systems.

The systems, compositions, and methods disclosed herein solve this and other problems in the art. For example, systems, compositions, and methods disclosed herein include both an argon gas blanket and an argon gas reservoir formed and/or throughout within the cosmetic formulation for replenishing the argon gas blanket and further preserving cosmetic formulations. This is a clear improvement in the art of cosmetic preservation. The selection of a heavy inert gas, such as argon, as opposed to any non-oxidizing gas favoured in the prior art, allows the formation of an inert gas blanket at the cosmetic-air interface that resists diffusion into atmospheric air upon opening the container. Further, any volumetric reduction in argon gas within the headspace due to use of the cosmetic formulation can be partially or completely replenished by the argon reservoir present within embodiments of the present disclosure.

In some of the disclosed embodiments, the argon gas reservoir is dispersed throughout the cosmetic formulation as a plurality of microbubbles. Additionally, or alternatively, the argon gas reservoir can be one or more of a chimney of argon gas disposed parallel to a longitudinal axis of the cosmetic formulation container or a stratified layer of argon gas disposed transverse to the longitudinal axis of the cosmetic formulation container. During use of the disclosed cosmetic formulations, the associated argon gas reservoir (or at least a portion thereof) is brought into fluid communication with the argon gas blanket and can act to replenish the argon gas blanket or, at the very least, displace atmospheric, oxygenated air from the adjacent cosmetic-air interface.

For example, after opening a cosmetic formulation container comprising a cosmetic formulation of the present disclosure, the user can, by a swiping motion, remove cosmetic from the container. As above, the argon gas blanket can be displaced by the user's finger and/or by the turbulence of the swiping motion. However, at least a portion of the argon gas reservoir of the cosmetic formulation can be brought into fluid communication with the argon gas blanket as a result of the cosmetic formulation being disrupted (e.g., by opening microbubbles containing argon gas), thereby replenishing the argon gas blanket and/or displace atmospheric, oxygenated air from an adjacent cosmetic-air interface to further preserve the cosmetic formulation.

The systems, methods, and compositions of the present disclosure also demonstrate a surprising and unexpected benefit of using argon-infused microbubbles.

For example, WO 2008/009617 to Karl Uwe Tapfer discussed in the Background, illustrates how microbubbles can positively impact physical, mechanical properties of a substance. In particular the Tapfer publication discloses the incorporation of microbubbles into ice creams, a type of organic formulation unrelated to cosmetics, to improve the physical, mechanical properties of the ice cream. As further provided in the Tapfer publication, a suspension of bubbles is incorporated throughout an ice cream mixture during manufacturing to impart a desirable scoopability, creaminess, and smooth texture sensation on the palate. The gas within the bubbles in Tapfer's publication acts as a physical insulator - not a chemical preservative - to resist multiple rounds of heat shock, and as such, the bubbles are disclosed as being made from any gas, namely nitrogen, oxygen, nitrogen dioxide, hydrogen, helium, or argon, without a distinction between oxygenated and inert gases.

The systems, methods, and compositions of the present disclosure beneficially demonstrate that argon-infused microbubbles can affect chemical properties of a cosmetic formulation (e.g., the oxidation state of organic compounds, resistance to microbial growth). As such, the use of an argon gas blanket and an argon gas reservoir as a means of chemically preserving cosmetic formulations provides additional improvements and advantages in the art.

As an example of our experience of preserving our cosmetic compositions with natural preservatives, such as Leucidal liquid radish root, grapefruit seed extract and rosemary, we have shown that these preservatives cannot be fully trusted to give sufficient preservation for a minimum of 36 months shelf life. During shelf life testing we observed fungal growth in - 8-25% of the samples (sample size > 300). By adding only argon to the head space this fungal growth percentage was lowered to between -2-18%. However, when we performed the compositions with natural preservatives according to this invention and injecting argon into the formula as a reservoir of microbubbles such as described here, the preservation became almost 100% secure, as fungal growth only occurred in less than 0.005% of samples over a 36 months designated shelf life.

The systems, methods, and compositions of the present disclosure beneficially demonstrate that by using together a natural preservative in a composition and argon-infused microbubbles can affect chemical properties of a cosmetic formulation (e.g., the oxidation state of organic compounds, resistance to microbial growth). As such, the use of an argon gas blanket and an argon gas reservoir together with a natural preservative, as means of chemically preserving cosmetic formulations provides additional improvements and advantages in the art. This is especially true when the formula including the argon reservoir bubbles is enclosed in a closed container; such as a cosmetic formulation container with a dispenser or a pump, which can be part of the system of the present invention.

For example, the argon gas blanket can be particularly effective against aerobic fungi within the container's headspace, and by enabling the argon gas blanket to be replenished through continued use of the cosmetic, the argon gas reservoir - in combination with the argon blanket - can act to prevent spoilage due to microbial growth and thereby preserve the cosmetic formulation for longer periods of time and/or increase its shelf life.

### Exemplary Systems for Preserving Cosmetic Formulations

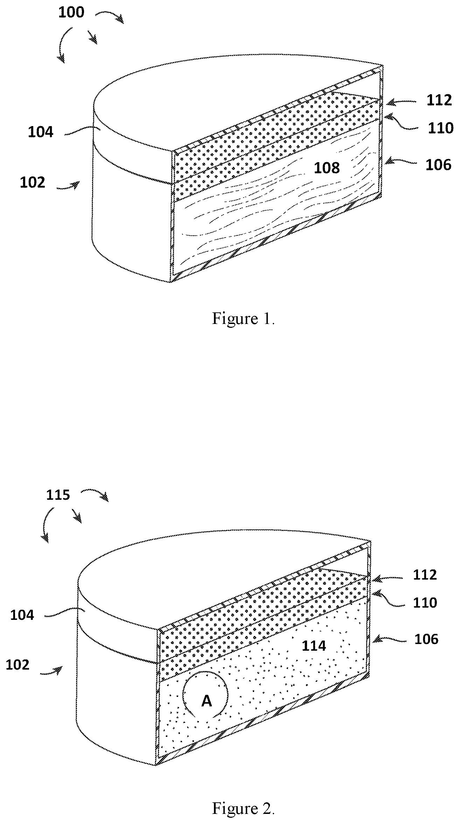

Referring now to the Figures, Figure 1 illustrates an exemplary system 100 for preserving cosmetic formulations. As shown, the system 100 includes a cosmetic formulation container 102 having a lid 104 that is selectively coupled to a vessel 106. The vessel 106 is sized and shaped to retain a defined volume of cosmetic formulation 108. The cosmetic formulation 108 includes a cosmetic-air interface 110, which as illustrated in Figure 1 is situated adjacent the headspace of the container 102.

In some embodiments, the headspace of the container 102 is filled with or includes an argon gas blanket 112. The argon gas blanket 112 preferably spans and rests upon the entire cosmetic-air interface 110, displacing any oxygenated air therefrom to create an anaerobic environment and thereby act to preserve the underlying cosmetic formulation 108.

Accordingly, preservation of the cosmetic formulation 108 includes, for example, preventing the oxidation of active ingredients, natural preservatives, or other compounds within the cosmetic formulation 108 and/or preventing the growth or survival of microbes at the cosmetic-air interface, within the cosmetic formulation 108, and/or within the headspace of the container 102. Preventing the growth or survival of microbes can additionally include for example, preventing the growth of aerobic or microaerophilic microbes, including aerobic and microaerophilic bacteria and fungi commonly associated with spoilage of cosmetic formulations (e.g., bacteria: Bacillus, spp., Staphylococcus spp., Streptococcus spp., Enterobacter spp., Pseudomonas spp., Escherichia coli Citrobacter freundi, and Klebsiella spp.; and fungi Aspergillus spp., Candida spp., Rhodotorula spp., and Penicillium spp.).

In some embodiments the contaminating microbe is a facultative anaerobe or capable of anaerobic respiration/fermentation. In such embodiments, the growth rate of the microbe may be hindered due to its growth in the presence of the argon gas, particularly compared to its growth rate in the absence of the argon gas. Accordingly, in such embodiments, the argon gas provides an increased shelf life and can beneficially prevent or at least limit the growth of microbes to preserve the cosmetic formulation for longer periods of time than would otherwise be possible without such treatment.

With continued reference to Figure 1, the vessel 106 can be incompletely filled with cosmetic formulation 108 (e.g., filled to a level below the upper edge of the vessel 106) such that the argon gas blanket 112 may rest on the cosmetic-air interface 110 and be surrounded by an interior sidewall of the vessel 106. The effect of this configuration is that at least a portion of the argon gas blanket 112 can remain substantially undisturbed and associated with the cosmetic-air interface 110 even when the lid 104 is uncoupled from the vessel 106.

Accordingly, the volume of the argon gas blanket can be proportional to the volume of space between the cosmetic-air interface and the upper edge of the vessel. In some embodiments, the volume of the argon gas blanket can be proportional to the total volume of available headspace in the container, although the volume of the argon gas blanket may be less than the total volume of headspace in the container. Preferably, the volume of the argon gas blanket is sufficient to cover at least 90%, of the cosmetic-air interface, or more preferably enough to cover the entire cosmetic-air interface to a depth of at least 1 mm but preferably at least 5 mm or more preferably at least 10 mm and yet more preferably the argon gas blanket fills initially the entire space between the cosmetic-air interface and the lid or other closure.

As discussed above, cosmetic formulations of the present disclosure can additionally include or be otherwise associated with an argon gas reservoir. Referring now to Figure 2, illustrated is a system 115 for preserving a cosmetic formulation that includes a container 102 substantially similar to the container of Figure 1. However, as shown in Figure 2, the cosmetic formulation 114 can additionally include a plurality of discrete pockets or bubbles formed therein and that are infused with argon gas. The discrete pockets or bubbles infused with argon gas can be collectively termed an argon gas reservoir such that the cosmetic formulation 114 is said to be associated with an argon gas reservoir for preserving the cosmetic formulation.

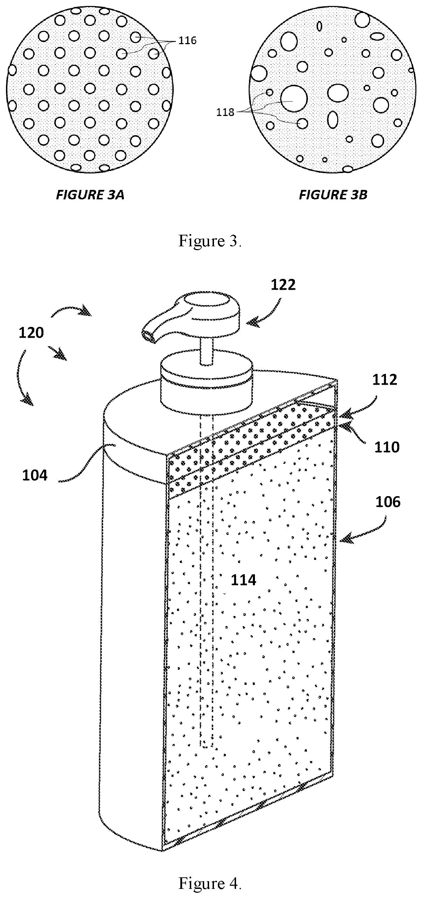

The discrete pockets or bubbles that form the argon gas reservoir can be distributed within the cosmetic formulation 114 in a number of ways. Two such examples are shown in Figures 3A and 3B, which illustrate a magnified, detail view of cross-sectional section "A" of Figure 2. As shown, the plurality of bubbles 116, 118 formed within the cosmetic formulation 114 can be uniformly distributed and uniformly sized (e.g., microbubbles 116 as shown in Figure 3A), or the plurality of bubbles formed within the cosmetic formulation 114 can be different sizes and non-uniformly distributed (e.g., microbubbles 118 as shown in Figure 3B). The plurality of bubbles may also be uniformly sized but non-uniformly distributed (not shown) or of non-uniform size but substantially uniformly distributed (not shown).

In an exemplary embodiment, the bubbles 116, 118 are microbubbles. That is, each of the bubbles 116 118 can have a diameter between about 10 µm - 1 mm. In some embodiments, the lower end of the range is selected from any of 10 µm, 20 µm, 30 µm, 40 µm, 50 µm, 60 µm, 70 µm, 80 µm, 90 µm, 100 µm, 125 µm, 150 µm, 175 µm, or 200 µm. It can also be selected from a range having endpoints selected from any of the foregoing. In an exemplary embodiment, the average diameter of microbubbles is no smaller than about 10 µm or preferably no smaller than about between about 50 µm.

Similarly, the upper end of the range can be selected from any of 500 µm, 600 µm, 700 µm, 800 µm, 850 µm, 900 µm, 950 µm, 975 µm, 1 mm, or any range having endpoints selected from any of the foregoing. In an exemplary embodiment, the average diameter of microbubbles can be no larger than about 1 mm or preferably no larger than about 500 µm.

**In** some embodiments, some or all of the bubbles 116, 118 may be smaller such that the bubbles 116, 118 form nanobubbles having diameters between about 10 nm - 1 µm.

The bubbles 116, 118 can also be a mixture of microbubbles and nanobubbles having diameters between about 10 nm - 5 mm or some (or all) of the bubbles can include larger bubbles or pockets having a diameter between about 1 mm - 5 mm.

It should be appreciated that the argon gas reservoir can include the same or similar total volume of argon gas infused into the cosmetic formulation, regardless of the volume of each bubble or discrete pocket. It may, however, be advantageous in some instances to have a larger number of discrete pockets or bubbles formed within the cosmetic formulation, as minor disruptions of the cosmetic formulation due to reiterated use are more likely to release argon gas from a plurality of smaller pockets and therefore have a higher likelihood of intermittently releasing argon gas to aid in the preservation of the cosmetic formulation (e.g., by maintaining the argon gas blanket on the cosmetic-air interface).

The bubbles can be formed throughout the entire cosmetic formulation, and argon gas can be released from the bubbles when the cosmetic formulation surrounding at least one side of the bubble is removed, opening the bubble to the surrounding environment. This can beneficially enable the preservation activity of the argon-gas-infused bubbles to have utility throughout the life or use of the cosmetic formulation.

For example, a cosmetic formulation having a cream-like consistency can include microbubbles distributed randomly throughout the cream. During manufacture or packaging, the microbubbles can be infused with an argon gas. When a user touches the creamy cosmetic formulation (e.g., by dipping their finger into the cosmetic formulation), the cosmetic formulation can adhere to and be removed by the user's touch. In doing so, the cosmetic formulation proximate the area that was touched is pulled or distorted in the direction of the touch, which can rupture microbubbles associated therewith and release the argon gas from within the ruptured microbubbles. The released argon gas can subsequently fuse with the argon gas blanket present at the cosmetic-air interface and/or be released onto an adjacent cosmetic-air interface, displacing oxygenated air from the adjacent interface.

In the foregoing and related examples, a larger volume of argon gas can be released when the cosmetic formulation includes a higher density of microbubbles versus when the cosmetic formulation includes a lower density of microbubbles. In a preferred embodiment, the density or number of microbubbles in a given formulation can be proportional to the surface area (e.g., the surface area of the cosmetic-air interface) to be covered. As in the example above, the density or number of microbubbles within each cubic millimeter of cosmetic formulation can be greater than one and less than or equal to five times greater than the surface area of each square millimeter. This way, the microbubbles surrounding or otherwise impacted/ruptured by the user's touch release a volume of argon gas that is sufficient to cover the surface area corresponding to the user s touch.

In a preferred embodiment, the inter gas reservoir constitutes argon. In some embodiments, argon can be combined with other gases having different densities, and this combination of argon-containing gases can be applied to the headspace of the container and/or to the bubbles and/or discrete pockets. The combination of argon-containing gases can flow across the cosmetic-air interface at different rates and can evacuate from bubbles and/or discrete pockets at different rates or with different tendencies. This may advantageously allow for better or more complete coverage of the cosmetic-air interface as motions or environmental stimuli (e.g., a gust of air) can differentially affect one of the argon-containing gases, dissipating it, while the other argon-containing gas(es) remain associated with the cosmetic-air interface.

The argon gas reservoir, whether alone or in combination with the argon gas blanket can preserve the associated cosmetic formulation from oxidation and/or microbial spoilage during storage and even after and/or during use by a consumer, as discussed above.

Referring now to Figure 4, illustrated is an additional exemplary embodiment of a system 120 for preserving a cosmetic formulation that, similar to Figure 2, can include a lid 104 selectively coupled to a vessel 106. The vessel 106 can include a cosmetic formulation 114 having a plurality of microbubbles formed therein that include or are infused with an argon gas, as described above.

The system 120 can additionally include a hand pump 122 for removing the cosmetic formulation 114 from the vessel 106. This can beneficially allow the user to access the contents of the vessel 106 without opening the lid 104. In such an embodiment, the argon gas stored within the headspace of the vessel 106 is less likely to be disrupted during each use as the system 120 remains closed.

In some embodiments, the system 120 is not airtight and allows air from the environment to enter the system 120 as cosmetic formulation 114 is ejected from the pump 122 ,thereby preventing a negative pressure from building up within the system 120.

In an exemplary use of the system 120 from Figure 4, the system 120 can be manufactured within an argon atmosphere where the headspace of the vessel 106 is filled with an argon gas blanket 112 and sealed with the lid 104. The container 120 may be stored and/or shipped with the headspace maintaining a blanket of argon gas 112 in association with the cosmetic-air interface 110. The argon gas blanket 112 formed within the headspace can act to preserve the cosmetic formulation 114 during storage and/or transit, as provided above.

A consumer can then receive the sealed system 120 and remove a desired portion of the cosmetic formulation 114 contained therein by operating the pump 122. As the cosmetic formulation 114 is drawn into the pump 122, argon gas can be released from the microbubbles (e.g., microbubbles 1 16, 118 described above) to maintain at least a portion of the surface of the cosmetic formulation 114 within the pump 122 in contact with argon gas. In this way, the argon gas blanket 112 in the headspace of the vessel 106 can protect/preserve the cosmetic formulation 114 at the cosmetic-air interface 110, and the disruption of discrete pockets of argon gas within the cosmetic formulation 114 can protect/preserve the cosmetic formulation 114 within the pump 122 and until used by the consumer.

### Exemplary Methods for Preserving Cosmetic Formulations

The following discussion now refers to a number of methods that may be performed. Although the method steps may be discussed in a certain order or illustrated in a flow chart as occurring in a particular order, no particular ordering is required unless specifically stated, or required because an act is dependent on another act being completed prior to the act being performed.

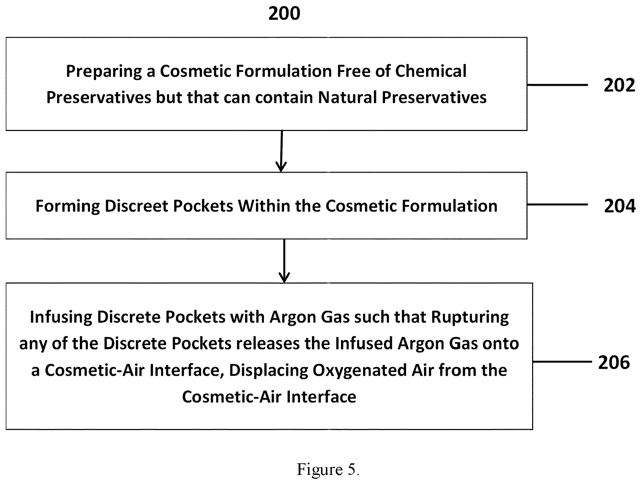

Figure 5 illustrates a method 200 of manufacturing a cosmetic formulation. The method 200 includes preparing a cosmetic formulation the cosmetic formulation being free of chemical preservatives (act 202). The cosmetic formulation can be prepared by any means known in the art and can include any desirable active ingredients, natural preservatives, or other compounds. In some embodiments, the cosmetic formulation includes certified-organic ingredients.

The cosmetic formulation may be any consistency known in the art, preferably a cream or gel-like consistency. If, for example, the cosmetic formulation has a cream or gel-like consistency, use may result in a divot forming within the cosmetic formulation.

In one variation of the method 200, the cosmetic formulation is prepared (act 202) in an argon gas environment (e.g., where atmospheric air is partially or completely replaced by an argon gas). This can beneficially act to prevent the oxidation of any compounds during the manufacturing process or to more easily facilitate loading argon gases into the headspace of the container during manufacturing.

The cosmetic formulations can be manufactured (e.g., according to method 200) and loaded into a vessel (e.g., vessel 106) from the top. Upon filling the vessel to the desired level, the lid can be coupled to the vessel, sealing it. The vessel is preferably pre-filled with argon gas so as to prevent any oxygenated air from being adsorbed to the surface of the vessel.

The method 200 additionally includes forming a plurality of discrete pockets within the cosmetic formulation (act 204) and infusing each of the plurality of discrete pockets with argon gas, wherein the discrete pockets are formed such that rupturing any of the plurality of discrete pockets releases the infused argon gas onto a cosmetic-air interface, displacing oxygenated air from the cosmetic-air interface (act 206). In some variations, acts 204 and 206 are performed simultaneously.

In an exemplary embodiment, the argon gas is infused within the discrete pockets as the discrete pockets are generated. For example, argon gas can be encapsulated within the cosmetic formulation with the assistance of gel forming compounds (e.g., xanthan gum, Arabic gum, or sclerotome gums).

In some embodiments, at or near the end of preparing the cosmetic formulation, any atmospheric air in the reaction chamber is vented and replaced by argon gas, and the gel forming compound is added to the cosmetic formulation under agitation (e.g., stirring). In a preferred embodiment the gel forming compound is added to the cosmetic formulation under regular atmospheric pressure (i.e., not under vacuum), and through stirring at a constant speed (e.g., 30 rpm - 1000 rpm), the gel forming compound creates discrete pockets of argon gas dispersed throughout the cosmetic formulation. In some embodiments, the mixing speed can affect the size and distribution of discrete pockets within the cosmetic formulation. For example, a higher, constant mixing speed can result in smaller pockets, whereas a variable speed can result in differently sized pockets.

As an additional example, the discrete pockets can be formed by streaming or bubbling the argon gas into the cosmetic formulation during generation of the cosmetic formulation or during or after filling of the containers. Additionally, or alternatively, the cosmetic formulation can be agitated while streaming or bubbling argon gas into the cosmetic formulation - forming argon-gas-infused pockets - or the cosmetic formulation may be agitated in an argon environment such that pockets infused with argon gas from the environment are captured within the cosmetic formulation.

As provided above, the cosmetic formulation may be manufactured and/or loaded into the vessel within an argon gas environment. In such instances, coupling the lid to the vessel traps argon gas within the headspace of the container. The formulation can, alternatively, be loaded into a vessel within a non-argon-gas (e.g., atmospheric air) environment and provided with an argon gas blanket before sealing the lid (e.g., through a narrow hose directed into the headspace of individual containers) or subsequent to sealing the lid (e.g., through a valve associated with the lid). In this case the vessel itself should preferably be pre-filled with argon gas prior to filling with the formulation, to displace any oxygenated air and/or moisture on the vessel walls.

In some variations of method 200, the cosmetic formulation is pumped into the container from the bottom. The container may be filled with a volume of argon gas prior to filling with cosmetic formulation. As the cosmetic formulation is gradually pumped into the container, the argon gas layer forms and remains on top of the cosmetic formulation, ensuring an oxygen-deprived environment surrounding the cosmetic formulation.

In some variations of method 200, the vessels are filled with argon gas, replacing all or substantially all of the oxygenated air from the vessel prior to filling with cosmetic formulation. This can be accomplished, for example, using individual narrow hoses that direct argon gas into each empty.

Alternatively, empty vessels can be filled with argon gas using an atmosphere exchange chamber where a number of containers (e.g., 100 - 500 or more) are inserted into the gas exchange chamber, and atmospheric exchange is monitored as argon gas is provided to the atmosphere exchange chamber through a series of openings on top of the chamber. The former atmosphere can be released through a hole (or series of holes) disposed at the bottom of the chamber. The application time is based on the size of the chamber and the selected pressure of argon gas entering the chamber.

In some variations of method 200, acts 204 and 206 include generating microbubbles within the cosmetic formulation. Stated another way, the discrete pockets disclosed above with respect to Figure 5 can be formed as microbubbles and may be formed using an argon gas source such that the resultant microbubbles are infused with argon gas upon formation. The size of the microbubbles formed can be adjusted and adapted to the manufacturer's specifications or as provided above and can result in uniformly (or non-uniformly) distributed pockets of uniform (or varying) sizes, discussed above.

The methods disclosed herein can enable stable preservation of associated chemical preservative-free cosmetic formulations for at least 12 months, preferably more than 18 months ,and most preferably more than 24 months on the shelf.

It should be appreciated that cosmetic formulations made by any of the foregoing methods are envisioned within the scope and content of this disclosure. Furthermore, any cosmetic formulation disclosed within the aforementioned systems are additionally, specifically included within the scope and content of this disclosure.

### Conclusion

Unless defined otherwise, all technical and scientific terms used herein have the same meaning as commonly understood by one of ordinary skill in the art to which the present disclosure pertains.

The present disclosure may be embodied in other specific forms without departing from its spirit or essential characteristics. The described embodiments are to be considered in all respects only as illustrative and not restrictive. The scope of the invention is, therefore, indicated by the appended claims rather than by the foregoing description. While certain embodiments and details have been included herein and in the attached disclosure for purposes of illustrating embodiments of the present disclosure, it will be apparent to those skilled in the art that various changes in the methods, products, devices, and apparatus disclosed herein may be made without departing from the scope of the disclosure or of the invention, which is defined in the appended claims. All changes which come within the meaning and range of equivalency of the claims are to be embraced within their scope.

### REFERENCES

Halla, N. et al. 2018. Cosmetics Preservation: A Review on Present Strategies. Molecules, 23, 1571; doi:10.3390/molecules23071571.
Clum, C. E. et. al (1993). Skin care compositions containing retinoids, US5,559,149.
Gurge, R. M. and M. W.Trumbore (2009) Foamable benzoyl peroxide composition for topical administration, US2010/0202978.
Stein, C. and A. Von Nieciecki, 1989. Verfaren zur sauerstoffarmen Abfüllung von oxidationsempfindlichen Lösungen, DE 3912153 C1.
Desrues, S. 2006. Method for conditioning a fluid production in a dispencer. US 2011/0146207 A1.

## Claims

1. A process for preparing and preserving a cosmetic formulation that is substantially without synthetic preservatives, comprising the steps of
preparing a cosmetic formulation that comprises a natural preservative, without conventional synthetic preservatives,
filling a container with argon gas,
forming an argon gas reservoir within the cosmetic formulation, wherein the argon gas reservoir is confined in compartments within the cosmetic formulation and comprises a plurality of bubbles dispersed throughout the cosmetic formulation,
introducing the cosmetic formulation and argon gas reservoir into said container, thereby displacing at least part of said argon gas occupying said container,
providing argon gas to the headspace above the cosmetic formulation to form an argon gas blanket, and
sealing the container with a closure, preferably performing the sealing in non-vacuum argon gas environment.

2. The process according to claim 1, wherein said cosmetic formulation is selected from the group consisting of a cream, gel, mousse, lotion, paste, foam, ointment, suspension, and emulsion.

3. The process according to claim 1, wherein said argon gas reservoir is configured to replenish the argon gas blanket when disrupted during use or extraction of the cosmetic formulation from the cosmetic formulation container.

4. The process according to claim 1, wherein said plurality of bubbles have an average diameter less than 1 mm, preferably between 10 µm - 1 mm, more preferably between 50 µm - 500 µm.

5. The process of claim 4, wherein forming the argon gas reservoir comprises infusing a plurality of microbubbles within the cosmetic formulation.

6. The process of claim 4 or 5, wherein forming the argon gas reservoir comprises stirring a gel forming compound into the prepared cosmetic formulation in an argon gas environment.

7. A cosmetic formulation that comprises a natural preservative and is without conventional synthetic preservatives and which contains an argon gas reservoir within the cosmetic formulation, wherein the argon gas reservoir is confined in compartments within the cosmetic formulation and comprises a plurality of bubbles dispersed throughout the cosmetic formulation, the formulation packaged in a container that is sealed with a headspace between the formulation and a closure of the container, wherein the headspace is filled with argon gas that forms an argon gas blanket above the cosmetic formulation.

8. The cosmetic formulation according to claim 7, which is selected from the group consisting of a cream, gel, mousse, lotion, paste, foam, ointment, suspension, and emulsion, and wherein the cosmetic formulation preferably comprises certified-organic ingredients.

9. The cosmetic formulation according to claim 7 or 8, wherein the plurality of bubbles dispersed throughout the cosmetic formulation have an average diameter less than about 1 mm, preferably between 10 µm - 1 mm, more preferably between 50 µm - 500 µm.

10. The cosmetic formulation according to any of claims 7 to 9, wherein the argon gas reservoir comprises a plurality of microbubbles dispersed throughout the cosmetic formulation, wherein said microbubbles are substantially uniform in size and have a substantially uniform distribution throughout the cosmetic formulation.

## Patentansprüche

1. Verfahren zur Herstellung und Konservierung einer kosmetischen Formulierung, die im Wesentlichen ohne synthetische Konservierungsmittel ist, umfassend die Schritte:
Herstellen einer kosmetischen Formulierung, die ein natürliches Konservierungsmittel umfasst, ohne herkömmliche synthetische Konservierungsmittel,
Füllen eines Behälters mit Argongas,
Bilden eines Argongasreservoirs innerhalb der kosmetischen Formulierung, wobei das Argongasreservoir in Kompartimenten innerhalb der kosmetischen Formulierung eingeschlossen ist und eine Vielzahl von Blasen umfasst, die in der gesamten kosmetischen Formulierung dispergiert sind,
Einbringen der kosmetischen Formulierung und des Argongasreservoirs in den Behälter, wodurch mindestens ein Teil des Argongases, das den Behälter einnimmt, verdrängt wird,
Bereitstellen von Argongas an den Kopfraum über der kosmetischen Formulierung, um eine Argongasdecke zu bilden, und
Versiegeln des Behälters mit einem Verschluss, vorzugsweise Durchführen des Versiegelns in einer Argongasumgebung ohne Vakuum.

2. Verfahren nach Anspruch 1, wobei die kosmetische Formulierung ausgewählt ist aus der Gruppe bestehend aus einer Creme, einem Gel, einer Mousse, einer Lotion, einer Paste, einem Schaum, einer Salbe, einer Suspension und einer Emulsion.

3. Verfahren nach Anspruch 1, wobei das Argongasreservoir ausgestaltet ist, um die Argongasdecke wieder aufzufüllen, wenn sie während Verwendung oder Extraktion der kosmetischen Formulierung aus dem Behälter für kosmetische Formulierung zerreißt.

4. Verfahren nach Anspruch 1, wobei die Vielzahl von Blasen einen mittleren Durchmesser von weniger als 1 mm, vorzugsweise zwischen 10 µm - 1 mm, besonders bevorzugt zwischen 50 µm - 500 µm aufweist.

5. Verfahren nach Anspruch 4, wobei Bilden des Argongasreservoirs Infundieren einer Vielzahl von Mikroblasen innerhalb der kosmetischen Formulierung umfasst.

6. Verfahren nach Anspruch 4 oder 5, wobei Bilden des Argongasreservoirs Rühren einer gelbildenden Verbindung in die hergestellte kosmetische Formulierung in einer Argongasumgebung umfasst.

7. Kosmetische Formulierung, die ein natürliches Konservierungsmittel umfasst und ohne herkömmliche synthetische Konservierungsmittel ist, und die ein Argongasreservoir innerhalb der kosmetischen Formulierung enthält, wobei das Argongasreservoir in Kompartimenten innerhalb der kosmetischen Formulierung eingeschlossen ist und eine Vielzahl von Blasen umfasst, die in der gesamten kosmetischen Formulierung dispergiert sind, wobei die Formulierung in einem Behälter verpackt ist, der mit einem Kopfraum zwischen der Formulierung und einem Verschluss des Behälters versiegelt ist, wobei der Kopfraum mit Argongas gefüllt ist, das eine Argongasdecke über der kosmetischen Formulierung bildet.

8. Kosmetische Formulierung nach Anspruch 7, die ausgewählt ist aus der Gruppe bestehend aus einer Creme, einem Gel, einer Mousse, einer Lotion, einer Paste, einem Schaum, einer Salbe, einer Suspension und einer Emulsion, und wobei die kosmetische Formulierung vorzugsweise zertifizierte organische Inhaltsstoffe umfasst.

9. Kosmetische Formulierung nach Anspruch 7 oder 8, wobei die Vielzahl von Blasen, die in der gesamten kosmetischen Formulierung dispergiert sind, einen mittleren Durchmesser von weniger als etwa 1 mm, vorzugsweise zwischen 10 µm - 1 mm, besonders bevorzugt zwischen 50 µm - 500 µm aufweist.

10. Kosmetische Formulierung nach einem der Ansprüche 7 bis 9, wobei das Argongasreservoir eine Vielzahl von Mikroblasen umfasst, die in der gesamten kosmetischen Formulierung dispergiert sind, wobei die Mikroblasen im Wesentlichen von gleichförmiger Größe sind und eine im Wesentlichen gleichförmige Verteilung in der gesamten kosmetischen Formulierung aufweisen.

## Revendications

1. Procédé destiné à préparer et conserver une formulation cosmétique qui est sensiblement dépourvue de conservateurs synthétiques, comprenant les étapes suivantes :
préparation d'une formulation cosmétique qui comprend un conservateur naturel, sans conservateurs synthétiques conventionnels,
remplissage d'un récipient avec de l'argon gazeux,
formation d'une réserve d'argon gazeux à l'intérieur de la formulation cosmétique, la réserve d'argon gazeux étant confinée dans des compartiments à l'intérieur de la formulation cosmétique et comprenant une pluralité de bulles dispersées dans toute la formulation cosmétique, introduction de la formule cosmétique et de la réserve d'argon gazeux à l'intérieur dudit récipient, ce qui permet de déplacer au moins une partie dudit argon gazeux occupant ledit récipient,
apport d'argon gazeux dans l'espace libre au-dessus de la formulation cosmétique pour former une couverture d'argon gazeux, et
scellement du récipient avec un dispositif de fermeture, en réalisant de préférence le scellement dans un environnement d'argon gazeux sans vide.

2. Procédé selon la revendication 1, dans lequel ladite formulation cosmétique est choisie dans le groupe constitué par une crème, un gel, une mousse, une lotion, une pâte, une mousse, une pommade, une suspension, et une émulsion.

3. Procédé selon la revendication 1, dans lequel ladite réserve d'argon gazeux est conçue pour reconstituer la couverture d'argon gazeux lorsqu'elle est perturbée pendant l'utilisation ou l'extraction de la formulation cosmétique du récipient de formulation cosmétique.

4. Procédé selon la revendication 1, dans lequel ladite pluralité de bulles ont un diamètre moyen inférieur à 1 mm, de préférence entre 10 µm et 1 mm, mieux encore entre 50 µm et 500 µm.

5. Procédé de la revendication 4, dans lequel la formation de la réserve d'argon gazeux comprend l'infusion d'une pluralité de microbulles à l'intérieur de la formulation cosmétique.

6. Procédé de la revendication 4 ou 5, dans lequel la formation de la réserve d'argon gazeux comprend l'agitation d'un composé formant un gel à l'intérieur de la formulation cosmétique préparée dans un environnement d'argon gazeux.

7. Formulation cosmétique qui comprend un conservateur naturel et est dépourvue de conservateurs synthétiques conventionnels et qui contient une réserve d'argon gazeux à l'intérieur de la formulation cosmétique, la réserve d'argon gazeux étant confinée dans des compartiments à l'intérieur de la formulation cosmétique et comprenant une pluralité de bulles dispersées dans toute la formulation cosmétique, la formulation étant conditionnée dans un récipient qui est scellé avec un espace libre entre la formulation et un dispositif de fermeture du récipient, l'espace libre étant rempli d'argon gazeux qui forme une couverture d'argon gazeux au-dessus de la formulation cosmétique.

8. Formulation cosmétique selon la revendication 7, qui est choisie dans le groupe constitué par une crème, un gel, une mousse, une lotion, une pâte, une mousse, une pommade, une suspension et une émulsion, la formulation cosmétique comprenant de préférence des ingrédients certifiés biologiques.

9. Formulation cosmétique selon la revendication 7 ou 8, la pluralité de bulles dispersées dans toute la formulation cosmétique ayant un diamètre moyen inférieur à environ 1 mm, de préférence entre 10 µm et 1 mm, mieux encore entre 50 µm et 500 µm.

10. Formulation cosmétique selon l'une quelconque des revendications 7 à 9, la réserve d'argon gazeux comprenant une pluralité de microbulles dispersées dans toute la formulation cosmétique, lesdites microbulles étant d'une taille sensiblement uniforme et présentant une distribution sensiblement uniforme dans toute la formulation cosmétique.
